# EUROPÄISCHE PATENTANMELDUNG

(11) **EP 0 621 481 A2**
(43) Veröffentlichungstag der Anmeldung: **26.10.1994**
(21) Anmeldenummer: 94105884.4
(22) Anmeldetag: 15.04.1994
(51) Int. Cl.: G01N 35/00, B01L 11/00

(54) **System zur Analyse von Inhaltsstoffen flüssiger Proben**

(30) Priorität: 23.04.1993 DE 4313253
(71) Anmelder: BOEHRINGER MANNHEIM GMBH, D-68298 Mannheim (DE)
(72) Erfinder: Schreiber, Joerg, Dr., D-68542 Heddesheim (DE); Schmid, Wilfried, Dr., D-68259 Mannheim (DE); Kuhr, Hans-Jürgen, Dr., D-68219 Mannheim (DE)

(57) **Zusammenfassung**

Die Erfindung betrifft ein System zur Analyse von Probenflüssigkeiten mittels Trockenreagenzien, das besonders für die Bestimmung klinischer Parameter geeignet ist. Zum System gehört ein Analysengerät, in dem sich Testelemente zur Durchführung eines analytischen Tests befinden. Die Form und Beschaffenheit der Testelemente ist der Verwendung im erfindungsgemäßen Analysensystem angepaßt. Das System ist besonders zur Durchführung von Analysen geeignet, für welche die zur Verfügung stehenden Testelemente im Kontakt mit der Umgebung eine geringe Lagerbeständigkeit aufweisen.

## Beschreibung

Die Erfindung betrifft ein System zur Analyse von Probenflüssigkeiten, das eine lineare Anordnung von Testelementen, eine Meßanordnung zur Detektierung von auf einem Testelement auftretenden Veränderungen und zwei oder mehr Testelemente innerhalb des Systems beinhaltet, wobei die Testelemente durch eine Vorrichtung gegen den Zutritt von Feuchte geschützt sind.

Die Erfindung beinhaltet ebenfalls Vorratsbehältnisse für zwei oder mehr Testelemente und Testelemente an sich, die aufgrund ihrer Formgebung gegen Feuchte abgedichtet werden können. Mit einem erfindungsgemäßen System ist ein Verfahren zur Analyse von Probenflüssigkeiten möglich, das ohne die Verwendung flüssiger Reagenzien arbeitet.

Im Stand der Technik sind Analysensysteme bekannt, die es gestatten, einzelne Parameter in Probenflüssigkeiten zu analysieren. Besonders im Bereich der Medizin und des Umweltschutzes existieren Systeme, die auch von wenig geschulten Personen bedient werden können. Einfach zu bedienen sind solche Geräte, die ohne die Verwendung von flüssigen Reagenzien arbeiten, was häufig auch als trockene Analyse bezeichnet wird. Beispielsweise sind für die Bestimmung von Glucose im Blut Geräte gebräuchlich, bei denen der Patient Blut auf einen Teststreifen aufgibt und die Messung in einem einfach zu bedienenden Gerät durchführt. Bei den im Stand der Technik gebräuchlichen Analysensystemen mit trockenen Reagenzien liegen die Testelemente separat außerhalb des Meßgerätes vor.

Bekannte Meßgeräte besitzen eine Öffnung, im allgemeinen einen Schlitz, in den Teststreifen eingeschoben werden können. Führungselemente stellen sicher, daß ein Teststreifen in der vorgesehenen Orientierung eingeschoben wird. Da der Teststreifen manuell in das Gerät eingeführt wird, müssen konstruktive Merkmale des Gerätes vorliegen, welche die gewünschte Positionierung des Teststreifens gewährleisten. Üblicherweise wird dies durch eine Begrenzung realisiert, die ein Einschieben über eine vorgegebene Zielposition verhindert. Die gebräuchlichsten Systeme verwenden analytische Tests, die auf Farbänderungen beruhen. Die Detektion der auf dem Teststreifen aufgetretenen Farbänderung kann reflexionsphotometrisch erfolgen. Eine Messung in Transmission ist ebenfalls möglich, setzt jedoch zumindest teilweise transparente Teststreifen voraus. Die für die Photometrie notwendigen Vorrichtungen zur Erzeugung und Detektion von Strahlung sind prinzipiell im Stand der Technik bekannt.

Teststreifen des Standes der Technik besitzen ein Testfeld und eine Halterung dieses Testfeldes, die eine Handhabung ermöglicht. Das Testfeld seinerseits kann aus mehreren Schichten bestehen. Es sind zum Beispiel für Bestimmungen im Blut Schichten von Vlies üblich, die eine Abtrennung der Zellen vom Serum bewirken. Außerdem sind Schichten üblich, in denen Reaktionen ablaufen oder die zu einer Dosierung des Analyten beitragen. Der mögliche Aufbau eines Teststreifens wird beispielsweise in der Patentanmeldung EP-A-0271854 beschrieben. Der Nachweis eines Analyten kann nur in wenigen Fällen durch die direkte Bildung eines Farbstoffes nachgewiesen werden, sondern es läuft in der Regel eine Kette von chemischen und/oder biochemischen Reaktionen ab, die in der Bildung oder Zersetzung eines Farbstoffes resultieren. Das Testfeld, auf dem der Farbstoff gebildet oder zersetzt wird, muß eine Größe und Formgebung aufweisen, die gewährleistet, daß auch bei Fertigungstoleranzen von Gerät und Testelement ein gleichbleibend großes Feld be- bzw. durchstrahlt werden kann. Im Stand der Technik bekannte Testelemente besitzen die Form eines Streifens oder einer flachen rechteckigen Platte. Als Materialien sind Papier, spezielle Pappen und Kunststoffe gebräuchlich. Das Testfeld ist entweder durch Tränken des Materials oder in Form von zusätzlichen Schichten auf die genannten Materialien aufgebracht. Die Lagerungsbeständigkeit vieler Testelemente wird durch die Einwirkung von Feuchtigkeit stark herabgesetzt, daher werden im Handel erhältliche Testelemente entweder einzeln eingesiegelt oder in größeren Mengen in speziellen Behältnissen verpackt zur Verfügung gestellt. Eine Versiegelung erfolgt in der Regel in kunststoff-laminierten Metallfolien, bevorzugt Aluminium-Laminaten. Diese Verpackungsform findet sich vor allem bei Schnelltests, die visuell ausgewertet werden. Zur Verwendung eines Testelements wird die Einsiegelung manuell aufgerissen und das Testelement entnommen. Teststreifen, die von ein und derselben Person wiederholt verwendet werden, z. B. bei der Glucosebestimmung in Blut, befinden sich im allgemeinen zu mehreren in Behältnissen aus feuchtigkeitsundurchlässigen Materialien, die zusätzlich noch Trockenmittel enthalten, um eingetretene Feuchtigkeit zu absorbieren.

Auch die Haltbarkeit von Tabletten wird durch Feuchtigkeit stark herabgesetzt. Behältnisse, die Trockenmittel enthalten, sind auch für Tabletten üblich, jedoch wird in diesem Bereich meist ein anderer Weg beschritten. Ein großer Teil der im Handel befindlichen Tabletten ist in sogenannte Blister eingesiegelt.

Der Meßvorgang mit bekannten Systemen zur Analyse mit trockenen Reagenzien erfolgt unter Verwendung eines Analysengerätes und separaten Teststreifen. Der Anwender des Systems öffnet manuell ein Vorratsbehältnis, entnimmt einen Teststreifen und verschließt das Vorratsgefäß, um im Gefäß verbliebene Teststreifen vor Feuchtigkeit zu schützen. Nachfolgend wird eine zu analysierende Flüssigkeit auf das Reaktionsfeld des Teststreifens aufgegeben. Der Teststreifen wird entweder direkt nach der Aufgabe oder nach dem Verstreichen einer Inkubationszeit in das Analysengerät eingeführt. Bei einigen bekannten Systemen erfolgt die Probenaufgabe erst, wenn sich der Teststreifen bereits im Gerät befindet. Bei beiden Vorgehensweisen findet die Positionierung des Teststreifens im Meßgerät durch den Anwender statt. Um eine genaue Positionierung zu gewährleisten, schiebt der Anwender den Teststreifen ein, bis ein mechanischer Widerstand spürbar wird.

Der Meßprozeß wird durch einen Tastendruck ausgelöst. Es existieren jedoch auch Systeme, bei denen der Meßvorgang durch das Einschieben des Teststreifens automatisch in Gang gesetzt wird. Nach dem Abschluß der Messung, die für den Anwender aufgrund einer Anzeige am Gerät zu erkennen ist, wird der Analysenprozeß durch die manuelle Entnahme des Teststreifens beendet.

Im Stand der Technik bekannte Systeme besitzen den Nachteil, daß Meßgerät und Teststreifen getrennt voneinander vorliegen. Der Benutzer muß aus diesem Grund neben dem eigentlichen Meßgerät mindestens ein weiteres Behältnis bei sich führen. Außerdem besitzen Vorratsgefäße für Teststreifen den Nachteil, daß alle Elemente mit Luftfeuchte in Berührung kommen, während ein Element entnommen wird. Ein weiterer Nachteil bestehender Systeme liegt in einer manuellen Positionierung des Teststreifens durch den Anwender, die eine Fehlbenutzung nicht vollständig verhindert.

Der Erfindung lag demnach die Aufgabe zugrunde, ein System zur Verfügung zu stellen, mit dem eine Anzahl von analytischen Bestimmungen durchgeführt werden können, ohne dem Meßgerät einzelne Teststreifen von außen zuzuführen. Die Bestimmung soll auch sicher erfolgen können, wenn das System in Gegenwart von Feuchtigkeit aufbewahrt wurde. Außerdem soll eine Positionierung von Testelementen mit Hilfe des Systems so erfolgen, daß Bedienungsfehler weitestgehend vermieden werden.

Erfindungsgemäß wurde diese Aufgabe durch ein System gelöst, das
- zwei oder mehr Testelemente, die in einer Reihe angeordnet sind,
- ein Vorratsbehältnis zur Aufnahme von Testelementen, das sich innerhalb des Systems befindet und aus dem System entnommen werden kann,
- eine Meßanordnung zur Detektierung von auf einem Testelement auftretenden Veränderungen und Andruckvorrichtungen zur Abdichtung von Testelementen gegen Zutritt von Feuchte, die auf das Vorratsbehältnis einen mechanischen Druck ausüben
besitzt.

Ein erfindungsgemäßes System beinhaltet ein Meßgerät und eine Anordnung von Testelementen. Das Meßgerät besitzt eine Einschubvorrichtung für ein Vorratsbehältnis, das zwei oder mehr Testelemente enthält. Die Testelemente können nacheinander an den Ort der Messung gebracht werden. Im Gerät befindet sich eine Meßvorrichtung, die eine Strahlungsquelle und einen Strahlungsempfänger beinhaltet. Eine Analyse kann durch detektierbare Signale erreicht werden, deren Signalstärke abhängig von der Konzentration des zu bestimmenden Parameters in der Probe ist. Dem Fachmann sind für Analysen geeignete detektierbare Signale, z. B. optischer, elektrischer oder magnetischer Art, bekannt. Bevorzugt sendet die Strahlungsquelle Licht im sichtbaren Teil des Spektrums aus. Es können Strahlungsquellen verwendet werden, die ein kontinuierliches oder ein diskretes Spektrum besitzen. Die ausgesandte Strahlung kann entweder direkt auf ein Testelement fallen oder zuvor durch eine optische Anordnung geführt werden. Eine erfindungsgemäße optische Anordnung kann Elemente wie Linsen, Spiegel und auch Bauteile enthalten, die zur Abschwächung von Bereichen der ausgesandten Strahlung dienen. Dies sind bevorzugt optische Filter, es sind jedoch auch Gitter oder Prismen möglich, die zu einer Selektion von bestimmten Frequenzen oder Frequenzbereichen führen.

Ein Teil der vom Testelement reflektierten Strahlung wird von dem Strahlungsempfänger aufgenommen, nachdem es eine optische Anordnung durchstrahlt hat. Eine solche optische Anordnung kann die weiter oben beschriebenen Elemente enthalten. Ebenfalls im Sinne der Erfindung ist es, wenn reflektierte Strahlung direkt auf den Strahlungsempfänger fällt. Der Strahlungsempfänger kann durch Photomultiplier, Photodioden, photovoltaische Elemente und andere im Stand der Technik bekannte Sensoren realisiert sein. Das vom Strahlungsempfänger abgegebene Signal kann entweder direkt oder nach Verarbeitung durch prinzipiell bekannte elektronische Schaltungen verarbeitet und mit einer Anzeigevorrichtung dargestellt werden. Eine erfindungsgemäße Anzeigevorrichtung kann z. B. ein analoges Meßinstrument oder bevorzugt ein digitales Display sein.

In das Gerät kann ebenfalls ein Lesegerät integriert sein, das Daten einliest, die für die verwendeten Testelemente und/oder die durchzuführende Analyse charakteristisch sind. Die Daten können beispielsweise auf einem Vorratsbehältnis für Testelemente vorliegen oder dem Gerät separat zugeführt werden. Als Lesegeräte sind Strichcodeleser, Magnetstreifenleser und andere bekannte Vorrichtungen möglich. Eingelesene Daten können sich z. B. auf die Art der Testelemente, Chargennummer, Verfallsdatum und auch herstellungsbedingt schwankende Parameter der Testelemente beziehen. Ebenfalls können sich die Daten auf die Art der durchzuführenden Analysen beziehen.

Zur Erfindung gehören Vorratsbehältnisse für zwei oder mehr Testelemente, die sowohl eine Vorrichtung zum Transport von Testelementen als auch eine Vorrichtung zum Abdichten von Testelementen gegen Feuchte beinhalten. Ein erfindungsgemäßes System kann beispielsweise mit Vorratsbehältnissen für zwei oder mehr Testelemente realisiert werden, die eine Öffnung besitzen, welche durch den Andruck von zwei gegenüberliegenden Seiten gegen Feuchtigkeit verschlossen werden kann. Für ein erfindungsgemäßes System ist es kennzeichnend, daß es ein Vorratsbehältnis besitzt, in dem Testelemente vor Feuchtigkeit geschützt aufbewahrt werden. Die Testelemente liegen in einer Reihe angeordnet vor und sind einzeln gegen Feuchte geschützt. Diese Einzelabdichtung kann erreicht werden, indem durch Andruck gegen jedes Testelement der Zutritt von Feuchte verhindert wird. Bevorzugt befindet sich diese Andruckmechanik innerhalb des Vorratsbehältnisses, so daß die Testelemente auch nach dem Entfernen des Vorratsbehältnisses aus dem System gegen Feuchte geschützt sind. Eine Andruckmechanik kann zum Beispiel mit Federn realisiert werden, die sich im System befinden und an deren einem Ende sich Körper befinden, die sich in eine Ausnehmung des Testelementes dichtend einpassen. Beispielsweise kann ein Testfeld, das sich in einer kreisrunden Ausnehmung eines Testelementes befindet, durch den Andruck einer Halbkugel verschlossen werden.

Außer der Möglichkeit, die Testelemente einzeln gegen Feuchte zu schützen, gibt es die Möglichkeit, das gesamte Vorratsbehältnis, in dem sie sich befinden, zu verschließen. Bevorzugt sind in diesem Fall solche Ausführungsformen, bei denen die Abdichtung durch Andruck auf die Gefäßöffnung erfolgt. Es ist daher vorteilhaft, wenn die Gefäßöffnung oder das gesamte Gefäß aus einem elastischen Material besteht, so daß die Gefäßöffnung auf einfache Weise zusammengedrückt werden kann. Bevorzugte Materialien sind Kunststoffe, besonders Polyvinylchlorid, Polyethylen, Polypropylen und Teflon. Es sind sowohl Vorratsbehältnisse möglich, die nach dem Verbrauch der Testelemente neu befüllt werden können als auch solche, die nach Entleerung verworfen werden. Der Transport von Testelementen aus dem Vorratsbehältnis an den Ort der Probenaufgabe kann zum einen durch eine Mechanik, z. B. einen Schieber erreicht werden, andererseits kann die Anordnung der Testelemente selbst zum Transport der Testelemente verwendet werden. Zu einer bevorzugten Ausführungsform der Erfindung gehört es ebenfalls, daß das Vorratsbehältnis dem System entnehmbar gestaltet ist.

Erfindungsgemäße Vorratsbehältnisse können ebenfalls eine zugehörige Kennzeichnung aufweisen, die sich entweder direkt an dem Vorratsbehältnis befindet oder separat vorliegt, jedoch eindeutig zuzuordnen ist. Ein Vorratsbehältnis kann beispielsweise einen Strichcode oder einen Magnetstreifen aufweisen, der beim Einschieben des Vorratsbehältnisses in das Gerät automatisch gelesen wird. In einer weiteren Ausführungsform liegen Vorratsbehältnis und Kennzeichnung zwar separat jedoch beispielsweise gemeinsam verpackt vor. Die Kennzeichnung kann dann in Form eines Streifens mit einem Strichcode, einer Chipkarte, einer radio-frequency identification card (RF-ID-Chip) und anderen in der Technik bekannten Speichermöglichkeiten vorliegen.

Ebenfalls gehört eine mechanisch zusammenhängende Anordnung von Testelementen zur Erfindung, die Sollbruchstellen enthält, so daß einzelne Testelemente herausgebrochen und entnommen werden können. Bei einer solchen zusammenhängenden Anordnung von Testelementen ist es möglich, daß ein Testelement nach seiner Benutzung z. B. durch Reißen, Brechen oder Abknicken von der Anordnung der übrigen Testelemente abgetrennt werden kann. Es ist ebenfalls eine mechanisch zusammenhängende Anordnung möglich, bei der die Testelemente ineinander verhakt sind, wie dies z. B. bei Teilen eines Puzzles der Fall ist. Diese Verbindung der Testelemente ermöglicht eine Bewegung der gesamten Anordnung von Testelementen durch Bewegung eines Testelementes, das sich außerhalb des Vorratsbehältnisses befindet. Ein Testelement außerhalb des Vorratsbehältnisses kann durch eine Bewegung senkrecht zur Transportrichtung aus der Anordnung gelöst werden. Bevorzugt befindet sich nach dem Abtrennen eines Testelementes der Griff oder Halter mindestens eines anderen Testelementes außerhalb des Vorratsbehältnisses, so daß dieses Testelement mit dem Griff oder Halter transportiert werden kann. Während sich der Griff oder Halter außerhalb des Vorratsgefäßes befindet, kann der feuchtigkeitsempfindliche Teil des betreffenden Testelementes innerhalb des Vorratsbehältnisses vor Feuchte geschützt werden.

Ebenfalls zur Erfindung gehören Testelemente, die eine solche Formgebung besitzen, daß durch den Andruck von Vorrichtungen eine Abdichtung des Testfeldes gegen Feuchtigkeit erreicht werden kann. Erfindungsgemäße Testelemente besitzen bevorzugt einen Rahmen aus einem Material, das mechanisch so stabil ist, daß es den Transport des Testelementes im erfindungsgemäßen System ermöglicht, ohne daß das Testfeld durch den Transport beschädigt wird. Bevorzugte Materialien sind Kunststoffe, Gläser, Pappen und Metalle. Der Rahmen besitzt eine Aussparung für das Testfeld und in bevorzugten Ausführungsformen kann er ebenfalls eine Formgebung besitzen, die seine Verwendung als Griff oder Halter ermöglicht. Der Rahmen ist mit dem Testfeld mechanisch verbunden, bevorzugt ist das Testfeld in einer Ebene durch den Rahmen umschlossen. Das Testfeld kann in den Rahmen gepreßt oder geklebt sein. Wird der Rahmen aus Kunststoff gefertigt, so ist es ebenfalls möglich, daß er während der Produktion um das Testfeld gegossen oder gespritzt wird. Das Testfeld beinhaltet gewünschtenfalls mehrere Schichten oder Bereiche, die zur Probenaufgabe dienen können, die gegebenenfalls Zellen abtrennen und in denen Reaktionen ablaufen, die bei Anwesenheit eines zu bestimmenden Analyten eine detektierbare Veränderung bewirken. Das Testfeld kann im übrigen Ausführungsformen wie weiter oben für den Stand der Technik beschrieben aufweisen. Das Testfeld kann erfindungsgemäß gegen Feuchte geschützt werden, indem Vorrichtungen im System zusammen mit dem Rahmen einen nach außen abgeschlossenen Raum bilden, in dem sich das jeweilige Testfeld befindet. Der Rahmen kann zu diesem Zweck beispielsweise planar sein oder auf seiner Oberfläche Erhebungen aufweisen, so daß das Testfeld beim Andruck einer Vorrichtung, bevorzugt einer Platte oder einem Stempel auf sowohl Ober- als auch Unterseite des Testelementes, abgeschlossen wird. Das Testelement kann ebenfalls so aufgebaut sein, daß sich das Testfeld in einer kreisförmigen Vertiefung des Rahmens befindet. In diesem Fall kann der Zutritt von Feuchte zum Testfeld verhindert werden, indem z.B. eine Kugel, eine Teilkugel oder ein konischer Zylinder auf die kreisförmige Vertiefung gedrückt wird.

Mit dem erfindungsgemäßen System ist ein Verfahren zur Analyse von Probenflüssigkeiten möglich, daß die Schritte umfaßt
- Transport eines Testelementes mit einem Schieber aus einem Vorratsbehältnis an den Ort der Messung
- Aufgabe der Probenflüssigkeit auf das Testelement
- Detektion einer probeninduzierten Veränderung des Testelementes
- Berechnung eines Meßergebnisses aus der detektierten Veränderung
- Ablesen des Meßergebnisses auf einer Anzeigevorrichtung
- Auswurf des Testelementes mit einem Greifarm oder Hebel.

Außerdem gehört ein Verfahren zur Analyse von Probenflüssigkeiten mit den Schritten
- Öffnen einer Dichtungsvorrichtung durch das Entlasten einer Andruckvorrichtung
- Transport eines Testelementes einer Anordnung von Testelementen aus einem Vorratsbehältnis an den Ort der Messung
- Schließen der Dichtungsvorrichtung
- Aufgabe von Probenflüssigkeit auf das Testelement
- Detektion einer probeninduzierten Veränderung des Testelementes
- Berechnung eines Meßergebnisses aus der detektierten Veränderung
- Ablesen des Meßergebnisses auf einer Anzeigevorrichtung
- Ablösen des gebrauchten Testelementes von der Anordnung der Testelemente
zur Erfindung.

Ein erfindungsgemäßes System bietet dem Benutzer den Vorteil, daß er kein externes Vorratsgefäß für Teststreifen benötigt, da ein Magazin mit Testelementen in das System integriert ist. Dies macht den bei bisherigen Systemen notwendigen manuellen Transport von einzelnen Teststreifen aus dem Vorratsbehältnis in das Gerät überflüssig, demnach wird auch eine Verschmutzungsgefahr z. B. durch ein Herunterfallen während des Transports vermieden. Außerdem können in einem erfindungsgemäßen System die Testelemente selbst nach Entfernen eines Testelements ausreichend vor Feuchte geschützt werden, so daß die Testelemente im Verlauf von Wochen oder Monaten ohne signifikanten Qualitätsverlust aufgebraucht werden können.

Eine besonders bevorzugte Ausführungsform eines erfindungsgemäßen Systems kann durch die im folgenden beschriebene Konstruktion realisiert werden.
- Figur 1:: Analysensystem
- Figur 2:: Analysensystem ohne Abdeckklappe bei geöffneter Schutzklappe
- Figur 3:: Vorratsbehältnis für Testelemente
- Figur 4:: Testelement
- Figur 5:: Analysensystem mit geschlossener Dichtungsvorrichtung
- Figur 6:: Vorratsbehältnisse für Testelemente mit Anordnungen von Testelementen
- Figur 7:: Analysensystem mit geöffneter Dichtungsvorrichtung

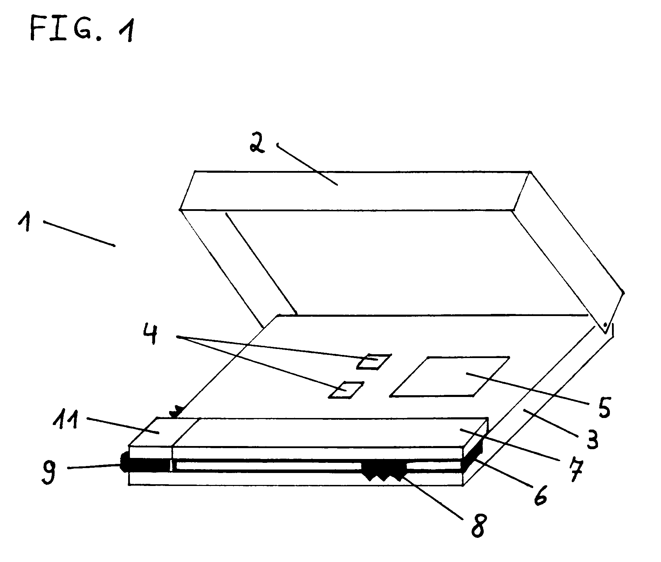

Figur 1 zeigt ein Analysensystem (1), bei dem die Abdeckklappe (2) geöffnet ist. Die Abdeckklappe (2) schützt den Grundkörper (3) des Systems vor Verschmutzung und Zerstörung. In den Grundkörper (3) sind Bedienungselemente (4) und eine Anzeigevorrichtung (5) integriert.

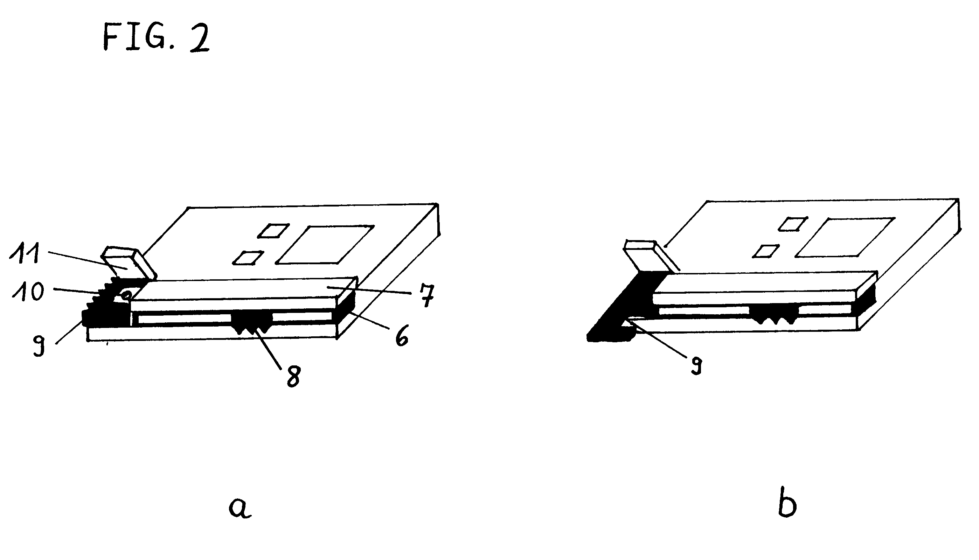

In der Figur 2 ist das System ohne die Schutzklappe (2) dargestellt. Das Vorratsbehältnis (6) für Testelemente befindet sich im vorderen Teil des Systems, unterhalb der Abdeckung (7); es besitzt in etwa die Form eines Riegels und erstreckt sich auf den größten Teil der Breite des Grundkörpers. Der Transport der Testelemente zum Ort der Probenaufgabe und Messung wird durch einen Schieber (8) vorgenommen. Probenaufgabe und Messung werden am gleichen Ort vorgenommen, der sich oberhalb der Meßöffnung (10) befindet. Unter dieser Meßöffnung (10) kann sich eine Optik oder wie weiter oben beschrieben direkt eine Strahlungsquelle und ein Empfangselement befinden. Während der Messung befindet sich ein Testelement oberhalb der Meßöffnung (10) und wird von einem Greifarm (9) umfaßt. Der Bereich oberhalb der Meßöffnung (10) kann durch eine Schutzklappe (11) verschlossen werden. Während der Messung ist somit ein Schutz gegen Licht aus der Umgebung möglich, der Fehlmessungen vermeidet. Bei Verwendung geeigneter Meßverfahren ist jedoch auch eine Messung ohne den Ausschluß von Umgebungslicht durch die Schutzklappe (11) möglich. Ist das System außer Betrieb, so kann der empfindliche Meßbereich mit der Schutzklappe (11) gegen Staub und Schmutz geschützt werden.

Die Durchführung einer Messung kann mit Hilfe der Figuren 2a und 2b erläutert werden. Ein mit Testelementen gefülltes Vorratsbehältnis (6) wird seitlich in das System eingeschoben. Der Schieber (8) wird auf die Meßöffnung (10) zubewegt, bis sich ein Testelement in der Aussparung des Greifarms (9) befindet. Nun wird die schutzklappe (11) geöffnet und Analytflüssigkeit auf das Testfeld des Testelementes aufgegeben. Zur Messung wird die Schutzklappe (11) geschlossen. Die Messung kann entweder durch eines der Bedienungselemente (4) in Gang gesetzt werden oder sie startet automatisch beim Schließen der Schutzklappe (11), z. B. gesteuert durch eine Lichtschranke oder einen Schalter, der von der Schutzklappe (11) betätigt wird. Das Ende der Messung kann auf der Anzeigevorrichtung (5) dargestellt oder durch eine Signal akustisch angezeigt werden. Das Meßergebnis wird auf der Anzeigevorrichtung (5) dargestellt und kann, falls gewünscht, im System abgespeichert werden. Ein verbrauchtes Testelement kann aus dem System entfernt werden, indem der Greifarm (9) in die in Figur 2b dargestellte Position gebracht wird. Das Testelement fällt automatisch aus dem Greifarm (9) heraus. Der Greifarm (9) wird nachfolgend wieder in die in Figur 2a dargestellte Position gebracht. Das System ist nun bereit für eine neue analytische Bestimmung.

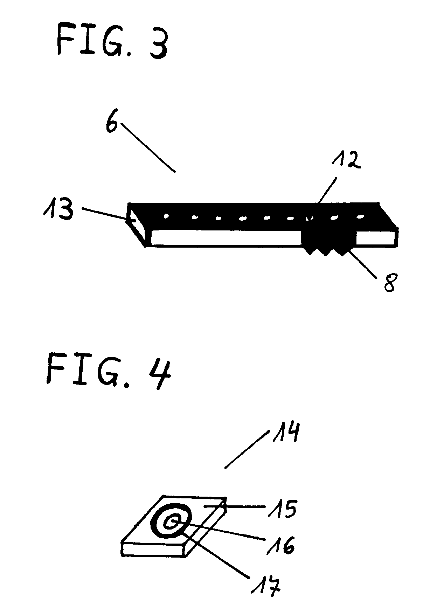

Figur 3 zeigt ein Vorratsbehältnis (6) für Testelemente. Auf der Oberseite sind eine Zahl von Erhebungen (12) sichtbar, auf die im Grundkörper (3) des Systems befindliche Kugeln drücken, um die Abdichtung der Testelemente zu erreichen. Unter jeder der Erhebungen (12) des Vorratsbehälters (6) befindet sich in gefülltem Zustand des Magazins ein Testelement. Das Vorratsbehältnis besitzt eine Austrittsöffnung (13) für Testelemente, die sich bei eingelegtem Vorratsbehältnis (6) neben der Meßöffnung (10) befindet.

In Figur 4 ist ein einzelnes Testelement (14) dargestellt. Das Testfeld (16) ist umgeben vom Rahmen (15), der auf Ober- und Unterseite eine kreisförmige Materialerhebung (17) besitzt, die eine Abdichtung gegen Feuchtigkeit ermöglicht, bzw. verbessert.

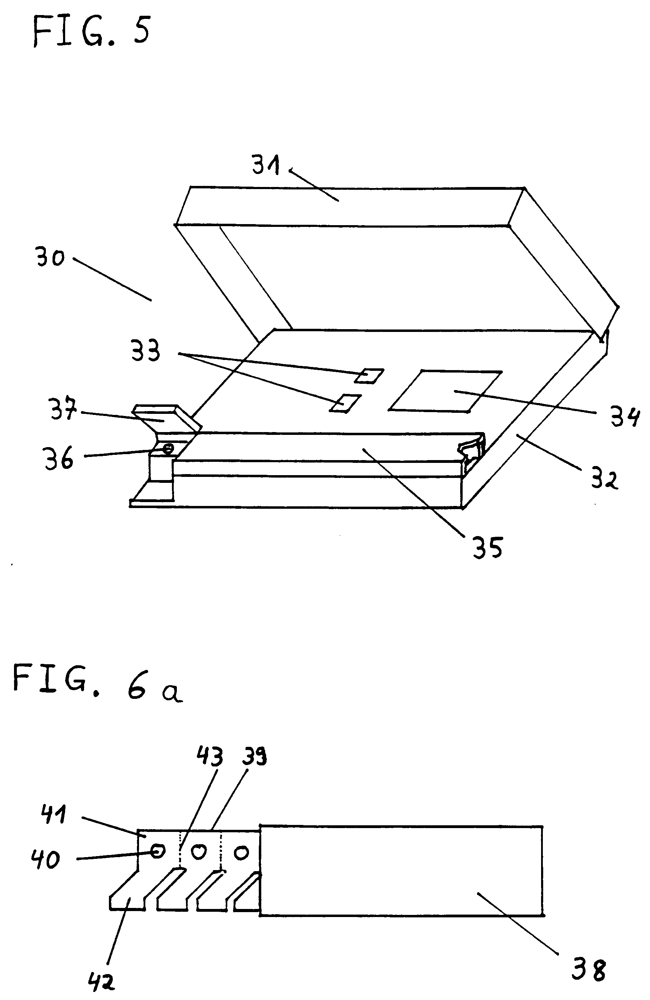

Eine weitere bevorzugte Ausführungsform eines erfindungsgemäßen Systems ist in Figur 5 dargestellt. Das System (30) besitzt eine Abdeckklappe (31) und einen Grundkörper (32). In den Grundkörper (32) integriert sind Bedienungselemente (33) und eine Anzeigevorrichtung (34). Das Vorratsbehältnis für Testelemente befindet sich unterhalb einer Dichtungsvorrichtung (35). Der Ort zur Messung und Probenaufgabe fällt auch bei dieser Ausführungsform zusammen und befindet sich oberhalb der Meßöffnung (36). Während der Messung wird das Testelement durch eine Schutzklappe (37) gegen Umgebungslicht geschützt.

Figur 6a zeigt ein Vorratsbehältnis (38) für Testelemente mit einer erfindungsgemäßen Anordnung von Testelementen (39). Das Vorratsbehältnis (38) besitzt die Form einer dünnen Schachtel, die lediglich eine Öffnung aufweist, in welche die Anordnung von Testelementen (39) eingeschoben werden kann. Die zur Anordnung der Testelemente (39) gehörigen Testelemente besitzen ein Testfeld (40), einen Rahmen (41) und einen Halter (42). Die Testelemente sind miteinander verbunden, es existieren jedoch Sollbruchstellen (43), an denen die Testelemente voneinander getrennt werden können.

Figur 6b zeigt ebenfalls ein erfindungsgemäßes Vorratsbehältnis (38) für Testelemente. Die Anordnung entspricht der in Figur 6a dargestellten, jedoch sind die Testelemente (41) ineinander verhakt. Ein Zapfen (44) greift in eine Aussparung (45). Die Testelemente (41) können aus dem Vorratsbehältnis (38) herausgezogen werden, ohne daß sie sich voneinander trennen. Befindet sich jedoch ein Testelement oberhalb der Meßöffnung (36), so bewegt es sich auf die Meßöffnung zu, wodurch die Verhakung aus Zapfen (44) und Aussparung (45) gelöst wird. Das letztgenannte Testelement kann nach der Messung dem System entnommen werden, ohne daß ein Abbrechen oder Abknicken notwendig ist.

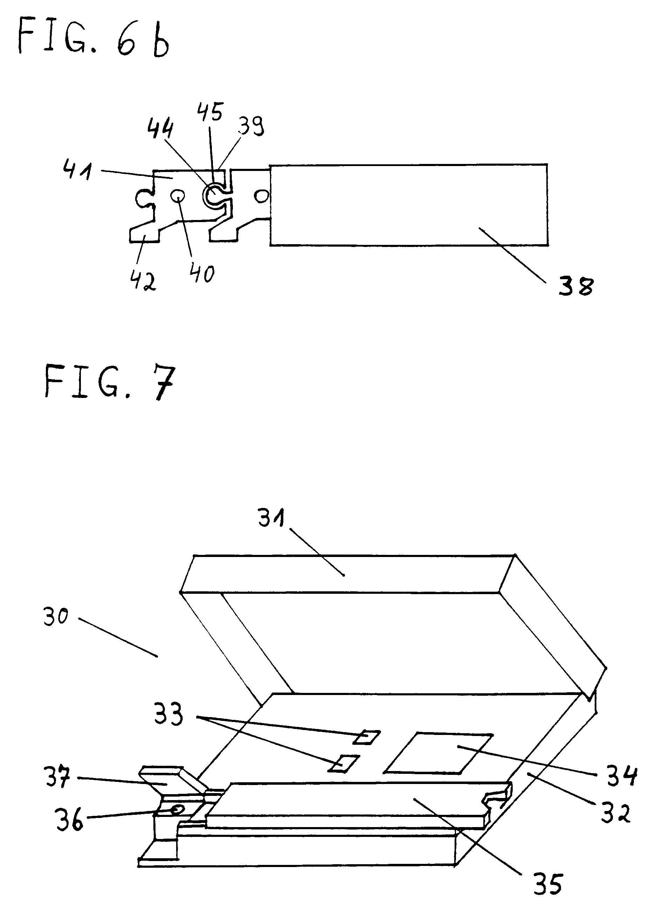

Mit Figur 7 kann die Funktionsweise der Dichtungsvorrichtung (35) erläutert werden. Soll eine neues Testelement in Meßposition gebracht werden, so wird zunächst die bajonettartig angeklemmte Dichtungsvorrichtung (35) aus der in Figur 5 dargestellten Position in die in Figur 7 dargestellte Position bewegt. Damit wird der mechanische Druck von der Öffnung des Vorratsbehältnisses genommen. Ein neues Testelement kann an seinem Halter (42) aus dem Vorratsbehältnis (38) gezogen werden. Wird die Dichtungsvorrichtung (35) in die in Figur 5 dargestellte Position zurückbewegt, so werden die Seiten des Vorratsbehältnisses (38) aufeinander gedrückt. Im Vorratsbehältnis (38) verbliebene Testelemente und insbesondere die Testfelder (40) dieser Testelemente werden durch die Dichtungsvorrichtung (35) gegen Feuchtigkeit abgedichtet.

Die Durchführung einer Messung mit dem in Figur 5 dargestellten System erfolgt in den folgenden Schritten:
- Öffnen der Dichtungsvorrichtung (35)
- Einschieben eines Vorratsbehältnisses (38) unter die Dichtungsvorrichtung (35)
- Herausziehen eines Testelementes an seinem Halter (42), bis sich das Testfeld (40) oberhalb der Meßöffnung (36) befindet
- Schließen der Dichtungsvorrichtung (35)
- Aufgabe von Probenflüssigkeit auf das Testfeld (40)
- Schließen der Schutzklappe (37)
- eventuell Betätigung der Bedienungselemente (33)
- Ablesen des Meßergebnisses von der Anzeigevorrichtung (34)
- Öffnen der Schutzklappe (37)
- Abbrechen des verbrauchten Testelementes an einer Sollbruchstelle (43) zwischen dem verbrauchten und dem nächsten neuen Testelement.
- Schließen der Schutzklappe (37)
Ist bereits ein Vorratsbehältnis in das System eingelegt, so kann der entsprechende Verfahrensschritt aus der obigen Auflistung entfallen.

Bei Verwendung geeigneter Meßverfahren, die im Stand der Technik bekannt sind, können aus der obigen Auflistung die Schritte zum Öffnen und Schließen der Schutzklappe (37) entfallen. Die Messung kann durch eine Betätigung der Bedienungselemente (33) oder durch Detektion der Probenaufgabe in Gang gesetzt werden.

### Bezugszeichenliste

- (1): System
- (2): Abdeckklappe
- (3): Grundkörper
- (4): Bedienungselemente
- (5): Anzeigevorrichtung
- (6): Vorratsbehältnis für Testelemente
- (7): Abdeckung
- (8): Schieber
- (9): Greifarm
- (10): Meßöffnung
- (11): Schutzklappe
- (12): Erhebungen
- (13): Öffnung des Vorratsbehältnisses
- (14): Testelement
- (15): Rahmen des Testelements
- (16): Testfeld
- (17): Materialerhebung des Testelements
- (30): System
- (31): Abdeckklappe
- (32): Grundkörper
- (33): Bedienungselemente
- (34): Anzeigevorrichtung
- (35): Dichtungsvorrichtung
- (36): Meßöffnung
- (37): Schutzklappe
- (38): Vorratsbehältnis für Testelemente
- (39): Anordnung von Testelementen
- (40): Testfeld
- (41): Rahmen des Testelementes
- (42): Halter
- (43): Sollbruchstelle
- (44): Zapfen eines Testelementes
- (45): Aussparung eines Testelementes

## Patentansprüche

1. System zur Analyse von Probenflüssigkeiten, beinhaltend
- zwei oder mehr Testelemente, die in einer Reihe angeordnet sind,
- Vorratsbehältnis zur Aufnahme von Testelementen, das sich innerhalb des Systems befindet und aus dem System entnommen werden kann,
- eine Meßanordnung zur Detektierung von auf einem Testelement auftretenden Veränderungen,
- Andruckvorrichtungen zur Abdichtung von Testelementen gegen den Zutritt von Feuchte, die auf das Vorratsbehältnis mechanischen Druck ausüben.

2. System nach Anspruch 1, dadurch gekennzeichnet, daß die Testelemente mechanisch nicht verbunden sind.

3. System nach Anspruch 1 oder 2, dadurch gekennzeichnet, daß die Testelemente durch einen Schieber an einen Ort zur Messung gebracht werden.

4. System nach einem der vorangehenden Ansprüche, bei dem der Zutritt von Feuchte zum Testfeld eines Testelements verhindert wird, indem zum System gehörende Andruckvorrichtungen auf zwei gegenüberliegende Seiten des Testelementes drücken.

5. System nach einem der vorangehenden Ansprüche, bei dem im Vorratsbehältnis befindliche Testelemente dadurch gegen Feuchte geschützt werden, daß die Öffnung des Vorratsbehältnisses durch eine Vorrichtung, die mechanischen Druck ausübt, verschlossen ist.

6. System gemäß Anspruch 4, bei dem das Vorratsbehältnis die zur Abdichtung der Testelemente gegen Feuchte notwendigen Vorrichtungen besitzt.

7. System nach Anspruch 1, dadurch gekennzeichnet, daß die Testelemente in einer mechanisch zusammenhängenden Anordnung vorliegen.

8. System nach Anspruch 7, bei dem die Anordnung der Testelemente Sollbruchstellen enthält, die eine Abtrennung einzelner Testelemente ermöglicht.

9. System nach Anspruch 7, bei dem die Testelemente ineinander verhakt sind.

10. System nach einem der vorangehenden Ansprüche, bei dem in einer Positionierung eines Testelementes sowohl Probenaufgabe als auch Messung erfolgen können.

11. System nach einem der vorangehenden Ansprüche, das eine Lesevorrichtung für Daten aufweist.

12. Vorratsbehältnis enthaltend zwei oder mehr Testelemente, das
- eine Vorrichtung zum Transport von Testelementen aus dem Inneren des Vorratsbehältnisses nach außen
- Andruckvorrichtungen zum Abdichten von Testelementen gegen Feuchte
beinhaltet.

13. Vorratsbehältnis für zwei oder mehr Testelemente, das eine Öffnung besitzt, die durch Andruck der Wände des Behältnisses an ein Testelement gegen Feuchtigkeit verschlossen werden kann.

14. Vorratsbehältnis für Testelemente nach Anspruch 12 oder 13, auf dem Daten gespeichert vorliegen.

15. Testelement, bei dem sich das Testfeld in einer Vertiefung befindet und der Rand der Ausnehmung die Form eines Kreisringes besitzt, der durch eine Teilkugel oder ein Kegelsegment gegen den Zutritt von Feuchte verschlossen werden kann.

16. Mechanisch zusammenhängende Anordnung von Testelementen, die Sollbruchstellen enthält, so daß einzelne Testelemente herausgebrochen und entnommen werden können.

17. Anordnung von Testelementen gemäß Anspruch 16, dadurch gekennzeichnet, daß jedes Testelement eine Region besitzt, die keine Testsubstanz enthält und als Griff dient.

18. Verfahren zur Analyse von Probenflüssigkeiten mit einem System gemäß den Ansprüchen 1 bis 3, mit den Schritten:
- Transport eines Testelementes mit einem Schieber aus einem Vorratsbehältnis an den Ort der Messung
- Aufgabe der Probenflüssigkeit auf das Testelement
- Detektion einer probeninduzierten Veränderung des Testelementes
- Berechnung eines Meßergebnisses aus der detektierten Veränderung
- Ablesen des Meßergebnisses auf einer Anzeigevorrichtung
- Auswurf des Testelementes mit einem Greifarm oder Hebel.

19. Verfahren zur Analyse von Probenflüssigkeiten mit den Schritten
- Öffnen einer Dichtungsvorrichtung durch das Entlasten einer Andruckvorrichtung
- Transport eines Testelementes einer Anordnung von Testelementen aus einem Vorratsbehältnis an den Ort der Messung
- Schließen der Dichtungsvorrichtung
- Aufgabe von Probenflüssigkeit auf das Testelement
- Detektion einer probeninduzierten Veränderung des Testelementes
- Berechnung eines Meßergebnisses aus der detektierten Veränderung
- Ablesen des Meßergebnisses auf einer Anzeigevorrichtung
- Ablösen des gebrauchten Testelementes von der Anordnung der Testelemente.

20. Verfahren nach einem der Ansprüche 18 oder 19, bei dem Daten eingelesen werden, die im System befindliche Testelemente betreffen.

21. Verfahren nach Anspruch 20, bei dem die Daten beim Einschieben eines Vorratsbehältnisses in das Meßgerät eingelesen werden.
